# EUROPEAN PATENT APPLICATION

(11) **EP 1 609 803 A1**
(43) Date of publication of application: **28.12.2005**
(21) Application number: 04724770.5
(22) Date of filing: 31.03.2004
(51) Int. Cl.: C07K 16/28, C12N 15/62, A61K 39/395, A61P 1/04, A61P 1/16, A61P 3/10, A61P 5/14, A61P 7/00, A61P 7/04, A61P 7/06, A61P 11/06, A61P 15/08, A61P 17/00, A61P 17/06, A61P 21/04, A61P 25/00, A61P 27/02, A61P 29/00, A61P 35/00, A61P 35/02, A61P 37/02

(54) **MODIFIED ANTIBODY AGAINST CD22 AND UTILIZATION THEREOF**

(30) Priority: 31.03.2003 JP 2003096950
(71) Applicant: CHUGAI SEIYAKU KABUSHIKI KAISHA, Tokyo, 115-8543 (JP)
(72) Inventor: Tsuchiya, Masayuki, c/o Chugai Seiyaku K.K., Gotenba-shi, Shizuoka 412-0038 (JP); Kimura, Naoki, c/o Chugai Seiyaku Kabushiki Kaisha, Niihari-gun, Ibaraki 300-4101 (JP); Fukuda, Tatsuya, c/o Chugai Seiyaku K.K., Gotenba-shi, Shizuoka 412- 0038 (JP)
(74) Representative: Vossius & Partner
(86) International application number: PCT/JP2004/004696
(87) International publication number: WO 2004/087763

(57) **Abstract**

CD22 diabodies, in which heavy-chain and light-chain variable regions are linked by a 5-mer linker, were produced based on known sequence information for two types of anti-CD22 antibodies. The two diabodies produced were analyzed for their activity of binding to lymphoma cells and inducing lymphoma cell death (apoptosis). As a result, both diabodies were revealed to bind to the B-lymphoma cell line, "Raji", and to have apoptosis-inducing activity towards Raji cells as well as towards another B-lymphoma cell line: Daudi cells. These results show that minibodies of antibodies that recognize CD22 can be used as apoptosis-inducing agents for tumor cells such as lymphoma cells.

SEQUENCE LISTING

<110> CHUGAI SEIYAKU KABUSHIKI KAISHA

<120> MODIFIED ANTIBODY AGAINST CD22 AND UTILIZATION THEREOF

<130> C1-A0305P

<150> JP 2003-96950

<151> 2003-03-31

<160> 36

<170> PatentIn version 3.1

<210> 1

<211> 260

<212> PRT

<213> Artificial

<220>

<223> an artificially synthesized peptide sequence

<400> 1

Met Glu Arg His Trp Ile Phe Leu Phe Leu Phe Ser Val Thr Ala Gly

1               5                   10                  15

Val His Ser Gln Val Gln Leu Gln Glu Ser Gly Ala Glu Leu Ser Lys
            20                  25                  30

Pro Gly Ala Ser Val Lys Met Ser Cys Lys Ala Ser Gly Tyr Thr Phe
        35                  40                  45

Thr Ser Tyr Trp Leu His Trp Ile Lys Gln Arg Pro Gly Gln Gly Leu
    50                  55                  60

Glu Trp Ile Gly Tyr Ile Asn Pro Arg Asn Asp Tyr Thr Glu Tyr Asn
65                  70                  75                  80

Gln Asn Phe Lys Asp Lys Ala Thr Leu Thr Ala Asp Lys Ser Ser Ser
                85                  90                  95

Thr Ala Tyr Met Gln Leu Ser Ser Leu Thr Ser Glu Asp Ser Ala Val
            100                 105                 110

Tyr Tyr Cys Ala Arg Arg Asp Ile Thr Thr Phe Tyr Trp Gly Gln Gly
        115                 120                 125

Thr Thr Leu Thr Val Ser Ser Gly Gly Gly Gly Ser Asp Ile Gln Leu
    130                 135                 140

Thr Gln Ser Pro Ser Ser Leu Ala Val Ser Ala Gly Glu Asn Val Thr

145                 150                 155                 160

Met Ser Cys Lys Ser Ser Gln Ser Val Leu Tyr Ser Ala Asn His Lys

                165                 170                 175

Asn Tyr Leu Ala Trp Tyr Gln Gln Lys Pro Gly Gln Ser Pro Lys Leu

            180                 185                 190

Leu Ile Tyr Trp Ala Ser Thr Arg Glu Ser Gly Val Pro Asp Arg Phe

        195                 200                 205

Thr Gly Ser Gly Ser Gly Thr Asp Phe Thr Leu Thr Ile Ser Arg Val

    210                 215                 220

Gln Val Glu Asp Leu Ala Ile Tyr Tyr Cys His Gln Tyr Leu Ser Ser

225                 230                 235                 240

Trp Thr Phe Gly Gly Gly Thr Lys Leu Glu Ile Lys Asp Tyr Lys Asp

                245                 250                 255

Asp Asp Asp Lys

            260

<210>  2

<211>  810

<212>  DNA
<213>  Artificial

<220>
<223>  an artificially synthesized DNA sequence

<220>
<221>  CDS
<222>  (14)..(799)
<223>

<400>  2
cctgaattcc acc atg gaa agg cac tgg atc ttt ctc ttc ctg ttt tca        49
               Met Glu Arg His Trp Ile Phe Leu Phe Leu Phe Ser
               1               5                   10

gta act gca ggt gtc cac tcc cag gtc cag ctg cag gag tca ggg gct        97
Val Thr Ala Gly Val His Ser Gln Val Gln Leu Gln Glu Ser Gly Ala
        15                  20                  25

gaa ctg tca aaa cct ggg gcc tca gtg aag atg tcc tgc aag gct tct       145
Glu Leu Ser Lys Pro Gly Ala Ser Val Lys Met Ser Cys Lys Ala Ser
    30                  35                  40

ggc tac acc ttt act agc tac tgg ctg cac tgg ata aaa cag agg cct       193
Gly Tyr Thr Phe Thr Ser Tyr Trp Leu His Trp Ile Lys Gln Arg Pro

45                  50                  55                  60

gga cag ggt ctg gaa tgg att gga tac att aat cct agg aat gat tat        241
Gly Gln Gly Leu Glu Trp Ile Gly Tyr Ile Asn Pro Arg Asn Asp Tyr
                65                  70                  75

act gag tac aat cag aac ttc aag gac aag gcc aca ttg act gca gac        289
Thr Glu Tyr Asn Gln Asn Phe Lys Asp Lys Ala Thr Leu Thr Ala Asp
            80                  85                  90

aaa tcc tcc agc aca gcc tac atg caa ctg agc agc ctg aca tct gag        337
Lys Ser Ser Ser Thr Ala Tyr Met Gln Leu Ser Ser Leu Thr Ser Glu
        95                  100                 105

gac tct gca gtc tat tac tgt gca aga agg gat att act acg ttc tac        385
Asp Ser Ala Val Tyr Tyr Cys Ala Arg Arg Asp Ile Thr Thr Phe Tyr
    110                 115                 120

tgg ggc caa ggc acc act ctc aca gtc tcc tcg ggt gga ggc ggt agc        433
Trp Gly Gln Gly Thr Thr Leu Thr Val Ser Ser Gly Gly Gly Gly Ser
125                 130                 135                 140

gac att cag ctg acc cag tct cca tca tct ctg gct gtg tct gca gga        481
Asp Ile Gln Leu Thr Gln Ser Pro Ser Ser Leu Ala Val Ser Ala Gly
                145                 150                 155

gaa aac gtc act atg agc tgt aag tcc agt caa agt gtt tta tac agt 529
Glu Asn Val Thr Met Ser Cys Lys Ser Ser Gln Ser Val Leu Tyr Ser
            160                 165                 170

gca aat cac aag aac tac ttg gcc tgg tac cag cag aaa cca ggg cag 577
Ala Asn His Lys Asn Tyr Leu Ala Trp Tyr Gln Gln Lys Pro Gly Gln
        175                 180                 185

tct cct aaa ctg ctg atc tac tgg gca tcc act agg gaa tct ggt gtc 625
Ser Pro Lys Leu Leu Ile Tyr Trp Ala Ser Thr Arg Glu Ser Gly Val
    190                 195                 200

cct gat cgc ttc aca ggc agc gga tct ggg aca gat ttt act ctt acc 673
Pro Asp Arg Phe Thr Gly Ser Gly Ser Gly Thr Asp Phe Thr Leu Thr
205                 210                 215                 220

atc agc aga gta caa gtt gaa gac ctg gca att tat tat tgt cac caa 721
Ile Ser Arg Val Gln Val Glu Asp Leu Ala Ile Tyr Tyr Cys His Gln
                225                 230                 235

tac ctc tcc tcg tgg acg ttc ggt gga ggg acc aag ctg gag atc aaa 769
Tyr Leu Ser Ser Trp Thr Phe Gly Gly Gly Thr Lys Leu Glu Ile Lys
            240                 245                 250

gac tac aag gat gac gac gat aag tga taa gcggccgcaa t 810
Asp Tyr Lys Asp Asp Asp Asp Lys

        255                 260

<210> 3

<211> 262

<212> PRT

<213> Artificial

<220>

<223> an artificially synthesized peptide sequence

<400> 3

Met Asn Phe Gly Leu Arg Leu Ile Phe Leu Val Leu Thr Leu Lys Gly

1               5                   10                  15

Val Lys Cys Glu Val Gln Leu Val Glu Ser Gly Gly Gly Leu Val Lys

            20                  25                  30

Pro Gly Gly Ser Leu Lys Leu Ser Cys Ala Ala Ser Gly Phe Ala Phe

        35                  40                  45

Ser Ile Tyr Asp Met Ser Trp Val Arg Gln Thr Pro Glu Lys Arg Leu

    50                  55                  60

Glu Trp Val Ala Tyr Ile Ser Ser Gly Gly Gly Thr Thr Tyr Tyr Pro

65                  70                  75                  80

Asp Thr Val Lys Gly Arg Phe Thr Ile Ser Arg Asp Asn Ala Lys Asn
                85                  90                  95
Thr Leu Tyr Leu Gln Met Ser Ser Leu Lys Ser Glu Asp Thr Ala Met
            100                 105                 110
Tyr Tyr Cys Ala Arg His Ser Gly Tyr Gly Ser Ser Tyr Gly Val Leu
        115                 120                 125
Phe Ala Tyr Trp Gly Gln Gly Thr Leu Val Thr Val Ser Ala Gly Gly
    130                 135                 140
Gly Gly Ser Asp Ile Gln Met Thr Gln Thr Thr Ser Ser Leu Ser Ala
145                 150                 155                 160
Ser Leu Gly Asp Arg Val Thr Ile Ser Cys Arg Ala Ser Gln Asp Ile
                165                 170                 175
Ser Asn Tyr Leu Asn Trp Tyr Gln Gln Lys Pro Asp Gly Thr Val Lys
            180                 185                 190
Leu Leu Ile Tyr Tyr Thr Ser Ile Leu His Ser Gly Val Pro Ser Lys
        195                 200                 205
Phe Ser Gly Ser Gly Ser Gly Thr Asp Tyr Ser Leu Thr Ile Ser Asn

210                 215                 220

Leu Glu Gln Glu Asp Phe Ala Thr Tyr Phe Cys Gln Gln Gly Asn Thr

225                 230                 235                 240

Leu Pro Trp Thr Phe Gly Gly Gly Thr Lys Leu Glu Ile Lys Asp Tyr

                245                 250                 255

Lys Asp Asp Asp Asp Lys

            260

<210>  4

<211>  816

<212>  DNA

<213>  Artificial

<220>

<223>  an artificially synthesized DNA sequence

<220>

<221>  CDS

<222>  (14)..(805)

<223>

<400>  4

cctgaattcc acc atg aac ttt ggg ctc aga ttg att ttc ctt gtc ctt 49
Met Asn Phe Gly Leu Arg Leu Ile Phe Leu Val Leu
1               5                   10

act tta aaa ggt gtg aag tgt gaa gtg cag ctg gtg gag tct ggg gga 97
Thr Leu Lys Gly Val Lys Cys Glu Val Gln Leu Val Glu Ser Gly Gly
        15                  20                  25

ggc tta gtg aag cct gga ggg tcc ctg aaa ctc tcc tgt gca gcc tct 145
Gly Leu Val Lys Pro Gly Gly Ser Leu Lys Leu Ser Cys Ala Ala Ser
    30                  35                  40

gga ttc gct ttc agt atc tat gac atg tct tgg gtt cgc cag act ccg 193
Gly Phe Ala Phe Ser Ile Tyr Asp Met Ser Trp Val Arg Gln Thr Pro
45                  50                  55                  60

gag aag agg ctg gag tgg gtc gca tac att agt agt ggt ggt ggt acc 241
Glu Lys Arg Leu Glu Trp Val Ala Tyr Ile Ser Ser Gly Gly Gly Thr
                65                  70                  75

acc tac tat cca gac act gtg aag ggc cga ttc acc atc tcc aga gac 289
Thr Tyr Tyr Pro Asp Thr Val Lys Gly Arg Phe Thr Ile Ser Arg Asp
            80                  85                  90

aat gcc aag aac acc ctg tac ctg caa atg agc agt ctg aag tct gag 337
Asn Ala Lys Asn Thr Leu Tyr Leu Gln Met Ser Ser Leu Lys Ser Glu

        95                  100                 105

gac aca gcc atg tat tac tgt gca aga cat agt ggc tac ggt agt agc 385
Asp Thr Ala Met Tyr Tyr Cys Ala Arg His Ser Gly Tyr Gly Ser Ser
    110                 115                 120

tac ggg gtt ttg ttt gct tac tgg ggc caa ggg act ctg gtc act gtc 433
Tyr Gly Val Leu Phe Ala Tyr Trp Gly Gln Gly Thr Leu Val Thr Val
125                 130                 135                 140

tct gca ggt gga ggc ggt agc gat atc cag atg acc cag act aca tcc 481
Ser Ala Gly Gly Gly Gly Ser Asp Ile Gln Met Thr Gln Thr Thr Ser
                145                 150                 155

tcc ctg tct gcc tct ctg gga gac aga gtc acc att agt tgc agg gca 529
Ser Leu Ser Ala Ser Leu Gly Asp Arg Val Thr Ile Ser Cys Arg Ala
            160                 165                 170

agt cag gac att agc aat tat tta aac tgg tat cag cag aaa cca gat 577
Ser Gln Asp Ile Ser Asn Tyr Leu Asn Trp Tyr Gln Gln Lys Pro Asp
        175                 180                 185

gga act gtt aaa ctc ctg atc tac tac aca tca ata tta cac tca gga 625
Gly Thr Val Lys Leu Leu Ile Tyr Tyr Thr Ser Ile Leu His Ser Gly
    190                 195                 200

gtc cca tca aag ttc agt ggc agt ggg tct gga aca gat tat tct ctc     673
Val Pro Ser Lys Phe Ser Gly Ser Gly Ser Gly Thr Asp Tyr Ser Leu
205                 210                 215                 220

acc att agc aac ctg gag caa gaa gat ttt gcc act tac ttt tgc caa     721
Thr Ile Ser Asn Leu Glu Gln Glu Asp Phe Ala Thr Tyr Phe Cys Gln
                225                 230                 235

cag ggt aat acg ctt ccg tgg acg ttc ggt gga ggc acc aag ctg gaa     769
Gln Gly Asn Thr Leu Pro Trp Thr Phe Gly Gly Gly Thr Lys Leu Glu
            240                 245                 250

atc aaa gac tac aag gat gac gac gat aag tga taa gcggccgcaa t        816
Ile Lys Asp Tyr Lys Asp Asp Asp Asp Lys
        255                 260

<210>  5
<211>  116
<212>  PRT
<213>  Artificial

<220>
<223>  an artificially synthesized peptide sequence

<400> 5

Gln Val Gln Leu Gln Glu Ser Gly Ala Glu Leu Ser Lys Pro Gly Ala

1               5                   10                  15

Ser Val Lys Met Ser Cys Lys Ala Ser Gly Tyr Thr Phe Thr Ser Tyr

            20                  25                  30

Trp Leu His Trp Ile Lys Gln Arg Pro Gly Gln Gly Leu Glu Trp Ile

        35                  40                  45

Gly Tyr Ile Asn Pro Arg Asn Asp Tyr Thr Glu Tyr Asn Gln Asn Phe

    50                  55                  60

Lys Asp Lys Ala Thr Leu Thr Ala Asp Lys Ser Ser Ser Thr Ala Tyr

65                  70                  75                  80

Met Gln Leu Ser Ser Leu Thr Ser Glu Asp Ser Ala Val Tyr Tyr Cys

                85                  90                  95

Ala Arg Arg Asp Ile Thr Thr Phe Tyr Trp Gly Gln Gly Thr Thr Leu

            100                 105                 110

Thr Val Ser Ser

        115

<210> 6

<211> 348

<212> DNA

<213> Artificial

<220>

<223> an artificially synthesized DNA sequence

<220>

<221> CDS

<222> (1)..(348)

<223>

<400> 6

cag gtc cag ctg cag gag tca ggg gct gaa ctg tca aaa cct ggg gcc 48

Gln Val Gln Leu Gln Glu Ser Gly Ala Glu Leu Ser Lys Pro Gly Ala

1               5                   10                  15

tca gtg aag atg tcc tgc aag gct tct ggc tac acc ttt act agc tac 96

Ser Val Lys Met Ser Cys Lys Ala Ser Gly Tyr Thr Phe Thr Ser Tyr

            20                  25                  30

tgg ctg cac tgg ata aaa cag agg cct gga cag ggt ctg gaa tgg att 144

Trp Leu His Trp Ile Lys Gln Arg Pro Gly Gln Gly Leu Glu Trp Ile

        35                  40                  45

gga tac att aat cct agg aat gat tat act gag tac aat cag aac ttc     192
Gly Tyr Ile Asn Pro Arg Asn Asp Tyr Thr Glu Tyr Asn Gln Asn Phe
    50                  55                  60

aag gac aag gcc aca ttg act gca gac aaa tcc tcc agc aca gcc tac     240
Lys Asp Lys Ala Thr Leu Thr Ala Asp Lys Ser Ser Ser Thr Ala Tyr
65                  70                  75                  80

atg caa ctg agc agc ctg aca tct gag gac tct gca gtc tat tac tgt     288
Met Gln Leu Ser Ser Leu Thr Ser Glu Asp Ser Ala Val Tyr Tyr Cys
                85                  90                  95

gca aga agg gat att act acg ttc tac tgg ggc caa ggc acc act ctc     336
Ala Arg Arg Asp Ile Thr Thr Phe Tyr Trp Gly Gln Gly Thr Thr Leu
            100                 105                 110

aca gtc tcc tcg                                                     348
Thr Val Ser Ser
        115

<210>  7
<211>  112
<212>  PRT
<213>  Artificial

<220>

<223>  an artificially synthesized peptide sequence

<400>  7

Asp Ile Gln Leu Thr Gln Ser Pro Ser Ser Leu Ala Val Ser Ala Gly
1               5                   10                  15

Glu Asn Val Thr Met Ser Cys Lys Ser Ser Gln Ser Val Leu Tyr Ser
            20                  25                  30

Ala Asn His Lys Asn Tyr Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Arg Val Gln Val Glu Asp Leu Ala Ile Tyr Tyr Cys His Gln
                85                  90                  95

Tyr Leu Ser Ser Trp Thr Phe Gly Gly Gly Thr Lys Leu Glu Ile Lys
            100                 105                 110

<210> 8

<211> 336

<212> DNA

<213> Artificial

<220>

<223> an artificially synthesized DNA sequence

<220>

<221> CDS

<222> (1)..(336)

<223>

<400> 8

gac att cag ctg acc cag tct cca tca tct ctg gct gtg tct gca gga 48

Asp Ile Gln Leu Thr Gln Ser Pro Ser Ser Leu Ala Val Ser Ala Gly

1               5                   10                  15

gaa aac gtc act atg agc tgt aag tcc agt caa agt gtt tta tac agt 96

Glu Asn Val Thr Met Ser Cys Lys Ser Ser Gln Ser Val Leu Tyr Ser

            20                  25                  30

gca aat cac aag aac tac ttg gcc tgg tac cag cag aaa cca ggg cag 144

Ala Asn His Lys Asn Tyr Leu Ala Trp Tyr Gln Gln Lys Pro Gly Gln

        35                  40                  45

tct cct aaa ctg ctg atc tac tgg gca tcc act agg gaa tct ggt gtc     192
Ser Pro Lys Leu Leu Ile Tyr Trp Ala Ser Thr Arg Glu Ser Gly Val
    50                  55                  60

cct gat cgc ttc aca ggc agc gga tct ggg aca gat ttt act ctt acc     240
Pro Asp Arg Phe Thr Gly Ser Gly Ser Gly Thr Asp Phe Thr Leu Thr
65                  70                  75                  80

atc agc aga gta caa gtt gaa gac ctg gca att tat tat tgt cac caa     288
Ile Ser Arg Val Gln Val Glu Asp Leu Ala Ile Tyr Tyr Cys His Gln
                85                  90                  95

tac ctc tcc tcg tgg acg ttc ggt gga ggg acc aag ctg gag atc aaa     336
Tyr Leu Ser Ser Trp Thr Phe Gly Gly Gly Thr Lys Leu Glu Ile Lys
            100                 105                 110

<210>  9
<211>  123
<212>  PRT
<213>  Artificial

<220>
<223>  an artificially synthesized peptide sequence

<400>  9

Glu Val Gln Leu Val Glu Ser Gly Gly Gly Leu Val Lys Pro Gly Gly
1               5                   10                  15

Ser Leu Lys Leu Ser Cys Ala Ala Ser Gly Phe Ala Phe Ser Ile Tyr
            20                  25                  30

Asp Met Ser Trp Val Arg Gln Thr Pro Glu Lys Arg Leu Glu Trp Val
        35                  40                  45

Ala Tyr Ile Ser Ser Gly Gly Gly Thr Thr Tyr Tyr Pro Asp Thr Val
    50                  55                  60

Lys Gly Arg Phe Thr Ile Ser Arg Asp Asn Ala Lys Asn Thr Leu Tyr
65                  70                  75                  80

Leu Gln Met Ser Ser Leu Lys Ser Glu Asp Thr Ala Met Tyr Tyr Cys
                85                  90                  95

Ala Arg His Ser Gly Tyr Gly Ser Ser Tyr Gly Val Leu Phe Ala Tyr
            100                 105                 110

Trp Gly Gln Gly Thr Leu Val Thr Val Ser Ala
        115                 120

<210> 10

<211>  369

<212>  DNA

<213>  Artificial

<220>

<223>  an artificially synthesized DNA sequence

<220>

<221>  CDS

<222>  (1)..(369)

<223>

<400>  10

gaa gtg cag ctg gtg gag tct ggg gga ggc tta gtg aag cct gga ggg      48

Glu Val Gln Leu Val Glu Ser Gly Gly Gly Leu Val Lys Pro Gly Gly

1               5                   10                  15

tcc ctg aaa ctc tcc tgt gca gcc tct gga ttc gct ttc agt atc tat      96

Ser Leu Lys Leu Ser Cys Ala Ala Ser Gly Phe Ala Phe Ser Ile Tyr

            20                  25                  30

gac atg tct tgg gtt cgc cag act ccg gag aag agg ctg gag tgg gtc      144

Asp Met Ser Trp Val Arg Gln Thr Pro Glu Lys Arg Leu Glu Trp Val

        35                  40                  45

gca tac att agt agt ggt ggt ggt acc acc tac tat cca gac act gtg      192

Ala Tyr Ile Ser Ser Gly Gly Gly Thr Thr Tyr Tyr Pro Asp Thr Val
    50                  55                  60

aag ggc cga ttc acc atc tcc aga gac aat gcc aag aac acc ctg tac 240
Lys Gly Arg Phe Thr Ile Ser Arg Asp Asn Ala Lys Asn Thr Leu Tyr
65                  70                  75                  80

ctg caa atg agc agt ctg aag tct gag gac aca gcc atg tat tac tgt 288
Leu Gln Met Ser Ser Leu Lys Ser Glu Asp Thr Ala Met Tyr Tyr Cys
                85                  90                  95

gca aga cat agt ggc tac ggt agt agc tac ggg gtt ttg ttt gct tac 336
Ala Arg His Ser Gly Tyr Gly Ser Ser Tyr Gly Val Leu Phe Ala Tyr
            100                 105                 110

tgg ggc caa ggg act ctg gtc act gtc tct gca 369
Trp Gly Gln Gly Thr Leu Val Thr Val Ser Ala
        115                 120

<210> 11
<211> 107
<212> PRT
<213> Artificial

<220>

<223>  an artificially synthesized peptide sequence

<400>  11

Asp Ile Gln Met Thr Gln Thr Thr Ser Ser Leu Ser Ala Ser Leu Gly
1               5                   10                  15

Asp Arg Val Thr Ile Ser Cys Arg Ala Ser Gln Asp Ile Ser Asn Tyr
            20                  25                  30

Leu Asn Trp Tyr Gln Gln Lys Pro Asp Gly Thr Val Lys Leu Leu Ile
        35                  40                  45

Tyr Tyr Thr Ser Ile Leu His Ser Gly Val Pro Ser Lys Phe Ser Gly
    50                  55                  60

Ser Gly Ser Gly Thr Asp Tyr Ser Leu Thr Ile Ser Asn Leu Glu Gln
65                  70                  75                  80

Glu Asp Phe Ala Thr Tyr Phe Cys Gln Gln Gly Asn Thr Leu Pro Trp
                85                  90                  95

Thr Phe Gly Gly Gly Thr Lys Leu Glu Ile Lys
            100                 105

<210>  12

<211>  321

<212>  DNA

<213>  Artificial

<220>

<223>  an artificially synthesized DNA sequence

<220>

<221>  CDS

<222>  (1)..(321)

<223>

<400>  12

gat atc cag atg acc cag act aca tcc tcc ctg tct gcc tct ctg gga        48

Asp Ile Gln Met Thr Gln Thr Thr Ser Ser Leu Ser Ala Ser Leu Gly

1               5                   10                  15

gac aga gtc acc att agt tgc agg gca agt cag gac att agc aat tat        96

Asp Arg Val Thr Ile Ser Cys Arg Ala Ser Gln Asp Ile Ser Asn Tyr

            20                  25                  30

tta aac tgg tat cag cag aaa cca gat gga act gtt aaa ctc ctg atc        144

Leu Asn Trp Tyr Gln Gln Lys Pro Asp Gly Thr Val Lys Leu Leu Ile

        35                  40                  45

tac tac aca tca ata tta cac tca gga gtc cca tca aag ttc agt ggc        192

Tyr Tyr Thr Ser Ile Leu His Ser Gly Val Pro Ser Lys Phe Ser Gly
    50                  55                  60

agt ggg tct gga aca gat tat tct ctc acc att agc aac ctg gag caa 240
Ser Gly Ser Gly Thr Asp Tyr Ser Leu Thr Ile Ser Asn Leu Glu Gln
65                  70                  75                  80

gaa gat ttt gcc act tac ttt tgc caa cag ggt aat acg ctt ccg tgg 288
Glu Asp Phe Ala Thr Tyr Phe Cys Gln Gln Gly Asn Thr Leu Pro Trp
                85                  90                  95

acg ttc ggt gga ggc acc aag ctg gaa atc aaa 321
Thr Phe Gly Gly Gly Thr Lys Leu Glu Ile Lys
            100                 105

<210> 13
<211> 88
<212> DNA
<213> Artificial

<220>
<223> an artificially synthesized DNA sequence

<400> 13
cctgaattcc accatggaaa ggcactggat ctttctcttc ctgttttcag taactgcagg 60

tgtccactcc caggtccagc tgcaggag                                          88

<210>  14
<211>  90
<212>  DNA
<213>  Artificial

<220>
<223>  an artificially synthesized DNA sequence

<400>  14
gatgtcctgc aaggcttctg gctacacctt tactagctac tggctgcact ggataaaaca      60

gaggcctgga cagggtctgg aatggattgg                                      90

<210>  15
<211>  87
<212>  DNA
<213>  Artificial

<220>
<223>  an artificially synthesized DNA sequence

<400>  15

cttcaaggac aaggccacat tgactgcaga caaatcctcc agcacagcct acatgcaact      60

gagcagcctg acatctgagg actctgc                                          87

<210>  16

<211>  88

<212>  DNA

<213>  Artificial

<220>

<223>  an artificially synthesized DNA sequence

<400>  16

ggcaccactc tcacagtctc ctcgggtgga ggcggtagcg acattcagct gacccagtct      60

ccatcatctc tggctgtgtc tgcaggag                                         88

<210>  17

<211>  91

<212>  DNA

<213>  Artificial

<220>

<223>  an artificially synthesized DNA sequence

<400>  17
cagtgcaaat cacaagaact acttggcctg gtaccagcag aaaccagggc agtctcctaa      60

actgctgatc tactgggcat ccactaggga a                                     91

<210>  18
<211>  105
<212>  DNA
<213>  Artificial

<220>
<223>  an artificially synthesized DNA sequence

<400>  18
ggcagcggat ctgggacaga ttttactctt accatcagca gagtacaagt tgaagacctg      60

gcaatttatt attgtcacca atacctctcc tcgtggacgt tcggt                     105

<210>  19
<211>  91
<212>  DNA
<213>  Artificial

<220>

<223>  an artificially synthesized DNA sequence

<400>  19

ggtgtagcca gaagccttgc aggacatctt cactgaggcc ccaggttttg acagttcagc      60

ccctgactcc tgcagctgga cctgggagtg g                                     91

<210>  20

<211>  96

<212>  DNA

<213>  Artificial

<220>

<223>  an artificially synthesized DNA sequence

<400>  20

tgcagtcaat gtggccttgt ccttgaagtt ctgattgtac tcagtataat cattcctagg      60

attaatgtat ccaatccatt ccagaccctg tccagg                                96

<210>  21

<211>  105

<212>  DNA

<213>  Artificial

<220>

<223>  an artificially synthesized DNA sequence

<400>  21

acccgaggag actgtgagag tggtgccttg gccccagtag aacgtagtaa tatcccttct      60

tgcacagtaa tagactgcag agtcctcaga tgtcaggctg ctcag                      105

<210>  22

<211>  102

<212>  DNA

<213>  Artificial

<220>

<223>  an artificially synthesized DNA sequence

<400>  22

ccaggccaag tagttcttgt gatttgcact gtataaaaca ctttgactgg acttacagct      60

catagtgacg ttttctcctg cagacacagc cagagatgat gg                         102

<210> 23

<211> 84

<212> DNA

<213> Artificial

<220>

<223> an artificially synthesized DNA sequence

<400> 23

aagagtaaaa tctgtcccag atccgctgcc tgtgaagcga tcagggacac cagattccct 60

agtggatgcc cagtagatca gcag 84

<210> 24

<211> 93

<212> DNA

<213> Artificial

<220>

<223> an artificially synthesized DNA sequence

<400> 24

attgcggccg cttatcactt atcgtcgtca tccttgtagt ctttgatctc cagcttggtc 60

cctccaccga acgtccacga ggagaggtat tgg 93

<210> 25

<211> 92

<212> DNA

<213> Artificial

<220>

<223> an artificially synthesized DNA sequence

<400> 25

cctgaattcc accatgaact ttgggctcag attgattttc cttgtcctta ctttaaaagg 60

tgtgaagtgt gaagtgcagc tggtggagtc tg 92

<210> 26

<211> 89

<212> DNA

<213> Artificial

<220>

<223> an artificially synthesized DNA sequence

<400> 26

gtgcagcctc tggattcgct ttcagtatct atgacatgtc ttgggttcgc cagactccgg 60

agaagaggct ggagtgggtc gcatacatt                                    89

<210>  27
<211>  86
<212>  DNA
<213>  Artificial

<220>
<223>  an artificially synthesized DNA sequence

<400>  27
gggccgattc accatctcca gagacaatgc caagaacacc ctgtacctgc aaatgagcag    60

tctgaagtct gaggacacag ccatgt                                         86

<210>  28
<211>  98
<212>  DNA
<213>  Artificial

<220>
<223>  an artificially synthesized DNA sequence

<400> 28

cggggttttg tttgcttact ggggccaagg gactctggtc actgtctctg caggtggagg 60

cggtagcgat atccagatga cccagactac atcctccc 98

<210> 29

<211> 114

<212> DNA

<213> Artificial

<220>

<223> an artificially synthesized DNA sequence

<400> 29

ttgcagggca agtcaggaca ttagcaatta tttaaactgg tatcagcaga aaccagatgg 60

aactgttaaa ctcctgatct actacacatc aatattacac tcaggagtcc catc 114

<210> 30

<211> 87

<212> DNA

<213> Artificial

<220>

<223>  an artificially synthesized DNA sequence

<400>  30
ctctcaccat tagcaacctg gagcaagaag attttgccac ttacttttgc caacagggta      60

atacgcttcc gtggacgttc ggtggag                                          87

<210>  31
<211>  91
<212>  DNA
<213>  Artificial

<220>
<223>  an artificially synthesized DNA sequence

<400>  31
ctgaaagcga atccagaggc tgcacaggag agtttcaggg accctccagg cttcactaag      60

cctcccccag actccaccag ctgcacttca c                                     91

<210>  32
<211>  91
<212>  DNA
<213>  Artificial

<220>

<223>  an artificially synthesized DNA sequence

<400>  32

gtctctggag atggtgaatc ggcccttcac agtgtctgga tagtaggtgg taccaccacc      60

actactaatg tatgcgaccc actccagcct c                                     91

<210>  33

<211>  90

<212>  DNA

<213>  Artificial

<220>

<223>  an artificially synthesized DNA sequence

<400>  33

ggccccagta agcaaacaaa accccgtagc tactaccgta gccactatgt cttgcacagt      60

aatacatggc tgtgtcctca gacttcagac                                       90

<210>  34

<211>  90

<212>  DNA
<213>  Artificial

<220>
<223>  an artificially synthesized DNA sequence

<400>  34
taattgctaa tgtcctgact tgccctgcaa ctaatggtga ctctgtctcc cagagaggca      60

gacagggagg atgtagtctg ggtcatctgg                                        90

<210>  35
<211>  93
<212>  DNA
<213>  Artificial

<220>
<223>  an artificially synthesized DNA sequence

<400>  35
tcttgctcca ggttgctaat ggtgagagaa taatctgttc cagacccact gccactgaac      60

tttgatggga ctcctgagtg taatattgat gtg                                   93

<210> 36

<211> 85

<212> DNA

<213> Artificial

<220>

<223> an artificially synthesized DNA sequence

<400> 36

attgcggccg cttatcactt atcgtcgtca tccttgtagt ctttgatttc cagcttggtg 60

cctccaccga acgtccacgg aagcg 85

## Description

### Technical Field

The present invention relates to minibodies of antibodies that recognize CD22.

### Background Art

CD22 is a molecule belonging to the Ig superfamily. CD22 is specifically expressed in B-cells, and is thus considered to function in suppressing signals from B-cell receptors. Furthermore, CD22 is known to be expressed in a variety of B-cell leukemia cells and malignant lymphoma cells in patients with hematopoietic disease. Since soluble CD22 has not been detected in serum, CD22-targeted antibody therapy might be possible (Non-Patent Documents 1 to 5).

Regarding the use of antibodies against CD22 in hematopoietic tumors, there are some reports showing the possibility of clinical use of humanized anti-CD22 antibodies for B-cell malignancies (Patent Documents 1 and 2). However, these documents do not disclose a relationship between anti-CD22 antibodies and apoptosis-inducing activity.

When anti-CD22 antibodies were chemically crosslinked using a crosslinking agent, a growth-suppressing effect was observed on the lymphoma cell line Daudi, whereas this effect was not observed in parent anti-CD22 antibodies alone (Non-Patent Document 6). In addition, this report makes no mention of apoptosis induction.

Minibodies are thought to have several advantages in clinical applications as compared to whole antibodies. Indeed, a method of apoptosis induction in tumor cells using crosslinked antibody structures, such as IgG or Fab'2 (without Fc fragments), against several kinds of antigens including CD22 has been disclosed (Patent Document 3). However, in this document, antibodies against CD22 have not actually been produced, and thus, their apoptosis-inducing activities have not been examined.

Therefore, there have been no reports of using anti-CD22 minibodies in this way to induce apoptosis of tumor cells.

The prior art literature relating to the invention of this application is shown below.
[Non-Patent Document 1] Nishii Kazuhiro, CURRENT THERAPY Vol. 20 No. 1 47-50
[Non-Patent Document 2] Tedder *et al*., Ann Rev Immunol 15:481-504 (1997)
[Non-Patent Document 3] Clark EA, J Immunol 150:4715-4718 (1993)
[Non-Patent Document 4] Sato *et al*., Immunity 5:551-562 (1996)
[Non-Patent Document 5] Li *et al*., Cell Immunol 118:85-99 (1993)
[Non-Patent Document 6] Ghetie *et al*., Proc. Natl. Acad. Sci. USA 94:7509-7514 (1997)
[Patent Document 1] Published Japanese Translation of International Publication No. 2001-518930
[Patent Document 2] Published Japanese Translation of International Publication No. Hei 10-505231
[Patent Document 3] WO 99/02567

### Disclosure of the Invention

A first objective of this invention is to provide minibodies converted from antibodies that recognize CD22. A further objective of this invention is to provide novel methods for treating hematopoietic tumors using these minibodies.

In order to generate minibodies against CD22, that is, to generate "CD22 diabodies" in which the variable regions of heavy- and light-chains are linked by a 5-mer amino acid linker, the present inventors first designed the nucleotide sequences of two CD22 diabodies using the previously reported sequences of two anti-CD22 antibodies, and synthesized them. (A Flag tag was included in the diabodies to facilitate purification.) Next, they inserted the cDNAs into animal cell expression vectors, respectively. After that, these vectors were transfected into DG44 or COS7 cells, and the diabodies produced in the culture supernatant were purified by anti-Flag M2 agarose affinity-purification.

Next, the present inventors examined the activity of two resultant diabodies in cell-binding and cell-death (apoptosis) induction in lymphoma cell lines. The results showed that both of these diabodies bind to cells of the B-lymphoma cell line "Raji", and that both have activity to induce apoptosis in Raji cells and Daudi cells, which are also a B-lymphoma cell line. These results show that minibodies of antibodies that recognize CD22 can be used as apoptosis-inducing agents against tumor cells such as lymphoma cells.

Specifically, the present invention provides following (1) to (12):
(1) A minibody that recognizes CD22.
(2) The minibody of claim 1, wherein the minibody is a diabody.
(3) A minibody of any one of (a) to (f):
   (a) a minibody comprising the amino acid sequence of SEQ ID NO: 1 or 3;
   (b) a minibody functionally equivalent to the minibody of (a), and comprising the amino acid sequence of SEQ ID NO: 1 or 3 wherein one or more amino acids are substituted, inserted, deleted, and/or added;
   (c) a minibody comprising the amino acid sequences of a CDR of SEQ ID NOs: 5 and 7;
   (d) a minibody functionally equivalent to the minibody of (c), and comprising the amino acid sequence of a CDR of SEQ ID NOs: 5 and 7 wherein one or more amino acids are substituted, inserted, deleted, and/or added;
   (e) a minibody comprising the amino acid sequences of a CDR of SEQ ID NOs: 9 and 11; and
   (f) a minibody functionally equivalent to the minibody of (c), and comprising the amino acid sequence of a CDR of SEQ ID NOs: 9 and 11 wherein one or more amino acids are substituted, inserted, deleted, and/or added.
(4) A method for producing a CD22-recognizing antibody with increased activity by converting a CD22-recognizing antibody to a low-molecular-weight antibody.
(5) The method of claim 4, wherein the conversion is conversion to a diabody.
(6) The method of claim 4 or 5, wherein the activity is an apoptosis-inducing activity.
(7) An apoptosis-inducing agent comprising the minibody of any one of claims 1 to 3 or the minibody produced by the method of any one of claims 4 to 6, as an active ingredient.
(8) The apoptosis-inducing agent of claim 7 that induces tumor cell apoptosis.
(9) The apoptosis-inducing agent of claim 8, wherein the tumor cell is a lymphoma or leukemic cell.
(10) An antitumor agent comprising the minibody of any one of claims 1 to 3 or the minibody produced by the method of any one of claims 4 to 6, as an active ingredient.
(11) The antitumor agent of claim 10, wherein the tumor is a blood tumor.
(12) The apoptosis-inducing agent of any one of claims 7 to 9, wherein the antibody is a diabody.
(13) The antitumor agent of claim 10 or 11, wherein the antibody is a diabody.

The present invention provides minibodies that recognize CD22. The minibodies of this invention are useful in that their activities are enhanced. Herein, the term "activities" refers to biological actions caused by antibody binding to antigens. Specific examples include apoptosis-inducing actions and anti-tumor actions.

The cells to be targeted by apoptosis-inducing action, anti-tumor action, and so on are not particularly limited, but tumor cells are preferable. Specific examples of the tumor cells are most preferably lymphoma cells and leukemia cells.

In the present invention, administration of minibodies that recognize CD22 can treat or prevent diseases such as tumors, including blood tumors (specifically, leukemia, myelodysplastic syndrome, malignant lymphoma, chronic myeloid leukemia, abnormal plasma cells (such as multiple myeloma or macroglobulinemia), myeloproliferative disease (such as polycythemia vera, essential thrombocythemia, or idiopathic myelofibrosis), or such), and autoimmune diseases (specifically, rheumatism, autoimmune hepatitis, autoimmune thyroiditis, autoimmune bullous diseases, autoimmune adrenal disease, autoimmune hemolytic anemia, autoimmune thrombocytopenic purpura, autoimmune atrophic gastritis, autoimmune neutropenia, autoimmune orchitis, autoimmune encephalomyelitis, autoimmune receptor disease, autoimmune infertility, Crohn's disease, systemic lupus erythematosus, multiple sclerosis, Basedow's disease, juvenile diabetes, Addison's disease, myasthenia gravis, lens-induced uveitis, psoriasis, and Behchet's disease).

In the present invention, CD22 refers to a molecule that belongs to the Ig superfamily, consists of seven Ig-like domains, and is genetically located at 19q13.1 (Tedder *et al*., Ann. Rev. Immunol. 15:481-504 (1997)).

In the present invention, a minibody comprises an antibody fragment lacking a portion of a whole antibody (for example, whole IgG). The minibodies of the present invention are not particularly limited, so long as they can bind an antigen. There are no particular limitations on the antibody fragments of the present invention, so long as they are portions of a whole antibody, and preferably contain a heavy chain variable region (VH) or a light chain variable region (VL). More preferably, the antibody fragments contain both a heavy chain variable region (VH) and a light chain variable region (VL). Specific examples of the antibody fragments include Fab, Fab', F(ab')2, Fv, and scFv (single chain Fv), but are preferably scFv (Huston, J. S. *et al*., Proc. Natl. Acad. Sci. U.S.A. (1988) 85, 5879-5883; Plickthun "The Pharmacology of Monoclonal Antibodies" Vol. 113, Resenburg and Moore Ed., Springer Verlag, New York, pp. 269-315, (1994)). Such antibody fragments can be prepared by treating an antibody with an enzyme, such as papain or pepsin for example, to generate antibody fragments, or by constructing genes that encode these antibody fragments, introducing them into expression vectors, and then expressing them in appropriate host cells (see, for example, Co, M. S. *et al*., 1994, J. Immunol. 152, 2968-2976; Better, M. and Horwitz, A. H., 1989, Methods Enzymol. 178, 476-496; Pluckthun, A. and Skerra, A., 1989, Methods Enzymol. 178, 497-515; Lamoyi, E., 1986, Methods Enzymol. 121, 652-663; Rousseaux, J. *et al*., 1986, Methods Enzymol. 121, 663-669; Bird, R. E. and Walker, B. W., 1991, Trends Biotechnol. 9, 132-137).

The minibodies of this invention preferably have smaller molecular weights than a whole antibody, however, they may form multimers including dimers, trimers, and tetramers, and their molecular weights may become greater than that of a whole antibody.

A preferred minibody of this invention is an antibody comprising two or more antibody VHs and two or more antibody VLs, in which each of these variable regions is linked directly or indirectly via linkers or such. Such linkages may be covalent bonds or non-covalent bonds, or may be both. An even more preferable minibody is an antibody comprising two or more VH-VL pairs formed by non-covalent bonds between VH and VL. In this case, the distance between one VH-VL pair and another VH-VL pair is preferably shorter in a minibody than in a whole antibody.

A particularly favorable minibody of this invention is a diabody. A diabody is a dimer formed by bonding two fragments, in which a variable region is linked to another variable region via a linker or such (for example, scFv) (hereinafter referred to as diabody-constituting fragments), and usually comprises two VLs and two VHs (P. Holliger *et al*., Proc. Natl. Acad. Sci. USA, 90, 6444-6448 (1993); EP404097; WO93/11161; Johnson *et al*., Method in Enzymology, 203, 88-98, (1991); Holliger *et al*., Protein Engineering, 9, 299-305, (1996); Perisic *et al*., Structure, 2, 1217-1226, (1994); John *et al*., Protein Engineering, 12(7), 597-604, (1999); Holliger *et al*., Proc. Natl. Acad. Sci. USA., 90, 6444-6448, (1993); Atwell *et al*., Mol. Immunol. 33, 1301-1312, (1996)). The bonds between the diabody-constituting fragments may be non-covalent or covalent, but are preferably non-covalent.

Alternatively, diabody-constituting fragments may be bound by a linker or such to form a single chain diabody (sc diabody). In such cases, linking the diabody-constituting fragments using a long linker of about 20 amino acids allows diabody-constituting fragments on the same chain to form a dimer via non-covalent bonds to each other.

Diabody-constituting fragments include those with a linked VL-VH, linked VL-VL, and linked VH-VH, and are preferably those with a linked VH-VL. In the diabody-constituting fragments, the linker used to link a variable region to a variable region is not particularly limited, but is preferably a linker short enough to prevent non-covalent bonding between variable regions in the same fragment. The length of such a linker can be appropriately determined by those skilled in the art, and is ordinarily 2 to 14 amino acids, preferably 3 to 9 amino acids, and most preferably 4 to 6 amino acids. In this case, linkers between a VL and VH encoded on the same fragment are short, and thus a VL and VH on the same strand do not form a non-covalent bond nor a single-chain V region fragment; rather, the fragment forms a dimer with another fragment via non-covalent bonding. Furthermore, according to the same principle as in diabody construction, three or more diabody-constituting fragments may be bonded to form multimeric antibodies, such as trimers and tetramers.

Without limitation, examples of the diabodies of this invention are shown below:
1. diabodies comprising the amino acid sequence of SEQ ID NO: 1 or 3;
2. diabodies functionally equivalent to a diabody comprising the sequence of SEQ ID NO: 1 or 3, and comprising the amino acid sequence of SEQ ID NO: 1 or 3 wherein one or more amino acids are mutated (substituted, deleted, inserted, and/or added);
3. diabodies comprising the amino acid sequences of the CDR (or variable region) of SEQ ID NO: 5 and the CDR (or variable region) of SEQ ID NO: 7;
4. diabodies functionally equivalent to a diabody comprising the sequences of the CDR (or variable region) of SEQ ID NO: 5 and the CDR (or variable region) of SEQ ID NO: 7, and comprising the amino acid sequences of the CDR (or variable region) of SEQ ID NO: 5 and the CDR (or variable region) of SEQ ID NO: 7 wherein one or more amino acids are mutated (substituted, deleted, inserted, and/or added);
5. diabodies comprising the amino acid sequences of the CDR (or variable region) of SEQ ID NO: 9 and the CDR (or variable region) of SEQ ID NO: 11; and
6. diabodies functionally equivalent to a diabody comprising the sequences of the CDR (or variable region) of SEQ ID NO: 9 and the CDR (or variable region) of SEQ ID NO: 11, and comprising the amino acid sequences of the CDR (or variable region) of SEQ ID NO: 9 and the CDR (or variable region) of SEQ ID NO: 11 wherein one or more amino acids are mutated (substituted, deleted, inserted, and/or added).

Herein, "functionally equivalent" means that the diabody of interest has an activity equivalent to an activity of a diabody comprising the sequence of SEQ ID NO: 1 or 3, a diabody comprising the sequences of the CDR (or variable region) of SEQ ID NO: 5 and the CDR (or variable region) of SEQ ID NO: 7, or a diabody comprising the sequences of the CDR (or variable region) of SEQ ID NO: 9 and the CDR (or variable region) of SEQ ID NO: 11 (for example, CD22 binding activity, and apoptosis-inducing activity).

The number of mutated amino acids is not particularly limited, but may ordinarily be 30 amino acids or less, preferably 15 amino acids or less, and more preferably five amino acids or less (for example, three amino acids or less). The amino acids are preferably mutated or modified in a way that conserves the properties of the amino acid side chain. Examples of amino acid side chain properties are: hydrophobic amino acids (A, I, L, M, F, P, W, Y, and V), hydrophilic amino acids (R, D, N, C, E, Q, G, H, K, S, and T), amino acids comprising the following side chains: aliphatic side chains (G, A, V, L, I, and P); hydroxyl-containing side chains (S, T, and Y); sulfur-containing side chains (C and M); carboxylic acid- and amide-containing side chains (D, N, E, and Q); basic side chains (R, K, and H); aromatic ring-containing side chains (H, F, Y, and W) (amino acids are represented by one-letter codes in parentheses). Polypeptides comprising a modified amino acid sequence, in which one or more amino acid residues is deleted, added, and/or replaced with other amino acids, are known to retain their original biological activity (Mark, D. F. *et al*., Proc. Natl. Acad. Sci. USA 81, 5662-5666 (1984); Zoller, M. J. & Smith, M. Nucleic Acids Research 10, 6487-6500 (1982); Wang, A. et al., Science 224, 1431-1433; Dalbadie-McFarland, G. *et al*., Proc. Natl. Acad. Sci. USA 79, 6409-6413 (1982)).

Furthermore, a diabody comprising the amino acid sequence of SEQ ID NO: 1 or 3, a diabody comprising the sequences of the CDR (or variable region) of SEQ ID NO: 5 and the CDR (or variable region) of SEQ ID NO: 7, or a diabody comprising the sequences of the CDR (or variable region) of SEQ ID NO: 9 and the CDR (or variable region) of SEQ ID NO: 11 may be humanized or chimerized to reduce heterologous antigenicity against humans.

In the amino acid sequence corresponding to the variable region of SEQ ID NO: 5, amino acids 31 to 35 correspond to CDR1, amino acids 50 to 66 correspond to CDR2, and amino acids 99 to 105 correspond to CDR3. In the amino acid sequence corresponding to the variable region of SEQ ID NO: 7, amino acids 24 to 40 correspond to CDR1, amino acids 56 to 62 correspond to CDR2, and amino acids 95 to 103 correspond to CDR3. In the amino acid sequence corresponding to the variable region of SEQ ID NO: 9, amino acids 31 to 35 correspond to CDR1, amino acids 50 to 66 correspond to CDR2, and amino acids 99 to 112 correspond to CDR3. In the amino acid sequence corresponding to the variable region of SEQ ID NO: 11, amino acids 24 to 34 correspond to CDR1, amino acids 50 to 56 correspond to CDR2, and amino acids 89 to 97 correspond to CDR3.

In the present invention, the CD22-recognizing minibodies specifically bind to CD22. They are not particularly limited, so long as they have a biological action. The minibodies of this invention can be prepared by methods well known to those skilled in the art. For example, as described in the Examples, the antibodies can be prepared based on a sequence of a CD22-recognizing antibody (particularly a variable region sequence or a CDR sequence), using genetic engineering techniques known to those skilled in the art.

A previously known antibody sequence can be used for the sequence of the CD22-recognizing antibody, or an anti- CD22 antibody can be prepared by a method well known to those skilled in the art using CD22 as the antigen, and then the sequence of this antibody can be obtained and used. Specifically, for example, this can be performed as follows: CD22 protein or its fragment is used as a sensitizing antigen to perform immunizations according to conventional immunization methods, the obtained immunocytes are fused with well-known parent cells according to conventional cell fusion methods, and monoclonal antibody-producing cells (hybridomas) are then screened by ordinary screening methods. Antigens can be prepared by known methods, such as methods using baculoviruses (WO98/46777 and such). Hybridomas can be prepared, for example, according to the method of Milstein *et al*. (Kohler, G and Milstein, C., Methods Enzymol. (1981) 73:3-46). When the antigen has low immunogenicity, immunization can be performed using the antigen bound to an immunogenic macromolecule, such as albumin. Thereafter, cDNAs of the variable region (V region) of the antibody are synthesized from the mRNAs of the hybridomas using reverse transcriptase, and the sequences of the obtained cDNAs can be determined by known methods.

Antibodies that recognize CD22 are not particularly limited, so long as they bind to CD22. Mouse antibodies, rat antibodies, rabbit antibodies, sheep antibodies, human antibodies, and such may be used as necessary. Alternatively, artificially modified, genetically recombinant antibodies, such as chimeric and humanized antibodies, may be used to reduce heterologous antigenicity against humans. These modified antibodies can be produced using known methods. A chimeric antibody is an antibody comprising the variable regions of the heavy and light chains of an antibody from a non-human mammal such as a mouse, and the constant regions of the heavy and light chains of a human antibody. A chimeric antibody can be produced by linking a DNA encoding the variable regions of a mouse antibody with a DNA encoding the constant regions of a human antibody, incorporating this into an expression vector, and then introducing the vector to a host.

Humanized antibodies are also referred to as "reshaped human antibodies". Such humanized antibodies are obtained by transferring the CDR of an antibody derived from a non-human mammal, for example a mouse, to the CDR of a human antibody, and general gene recombination procedures for this are also known. Specifically, a DNA sequence designed to link a murine antibody CDR to the framework region (FR) of a human antibody can be synthesized by PCR, using primers prepared from several oligonucleotides containing overlapping portions of terminal regions. The obtained DNA is linked to a DNA encoding human antibody constant regions, and this is then integrated into an expression vector, and the antibody is produced by introducing this vector into host cells (see European Patent Application EP 239400, and International Patent Application WO 96/02576). The human antibody FR to be linked via the CDR is selected so the CDR forms a favorable antigen-binding site. To form a suitable antigen-binding site, amino acids in the framework region of the antibody variable region may be substituted in the CDR of the reshaped human antibody, as necessary (Sato, K. *et al*., 1993, Cancer Res. 53, 851-856).

These chimeric antibodies and humanized antibodies can be chimerized, humanized, and such after their molecular weight is reduced, or their molecular weight can be reduced after they have been chimerized, humanized, or such.

Methods for obtaining human antibodies are also known. For example, human lymphocytes can be *sensitized in vitro* with a desired antigen, or with cells expressing the desired antigen, and the sensitized lymphocytes can be fused with human myeloma cells, such as U266, to obtain the desired human antibody with antigen-binding activity (Examined Published Japanese Patent Application No. (JP-B) Hei 1-59878). Further, a desired human antibody can be obtained by using a desired antigen to immunize transgenic animals that have a full repertoire of human antibody genes (see International Patent Application WO 93/12227, WO 92/03918, WO 94/02602, WO 94/25585, WO 96/34096, and WO 96/33735). Furthermore, techniques for obtaining human antibodies by panning using a human antibody library are also known. For example, variable regions of human antibodies can be expressed as single chain antibodies (scFvs) on the surface of phages using phage display methods, and phages that bind to antigens can be selected. The DNA sequences that encode the variable regions of human antibodies that bind an antigen can be determined by analyzing the genes of the selected phages. By determining the DNA sequences of the scFvs that bind to the antigens, appropriate expression vectors into which relevant sequences are inserted can be produced to yield human antibodies. These methods are already known, and are detailed in the following publications: WO 92/01047, WO 92/20791, WO 93/06213, WO 93/11236, WO 93/19172, WO 95/01438, and WO 95/15388.

The antibodies of this invention may be conjugated antibodies that are bonded to various molecules, such as polyethylene glycol (PEG), radioactive substances, and toxins. Such conjugate antibodies can be obtained by performing chemical modifications on the obtained antibodies. Methods for antibody modification are established in this field. The term "antibody" in this invention includes such conjugate antibodies.

The present invention includes DNAs that encode the antibodies of this invention. This invention also includes DNAs encoding antibodies that hybridize under stringent conditions to the aforementioned DNAs, and have antigen-binding capacity and activity. Hybridization techniques (Sambrook, J. *et al*., Molecular Cloning 2nd ed., 9.47-9.58, Cold Spring Harbor Lab. press, 1989) are well known to those skilled in the art, and hybridization conditions can be selected appropriately by those skilled in the art. Such hybridization conditions include, for example, conditions of low stringency. Examples of conditions of low stringency include post-hybridization washing in 0.1x SSC and 0.1% SDS at 42°C, and preferably in 0.1x SSC and 0.1% SDS at 50°C. More preferable hybridization conditions include those of high stringency. Highly stringent conditions include, for example, washing in 5x SSC and 0.1% SDS at 65°C. In these conditions, the higher the temperature, the higher the expectation of efficiently obtaining DNAs with a high homology. However, several factors, such as temperature and salt concentration, can influence hybridization stringency, and those skilled in the art can suitably select these factors to achieve similar stringencies.

The DNAs of this invention are used *for in vivo* and *in vitro* production of the antibodies of this invention, and for other applications, such as gene therapy. The DNAs of this invention may be in any form, so long as they encode the antibodies of this invention. More specifically, they may be cDNAs synthesized from mRNAs, genomic DNAs, chemically synthesized DNAs, or such. Furthermore, the DNAs of this invention include any nucleotide sequence based on the degeneracy of the genetic code, so long as they encode the antibodies of this invention.

The antibodies of this invention can be produced by methods well known to those skilled in the art. More specifically, a DNA of an antibody of interest is incorporated into an expression vector. In so doing, the DNA is incorporated into the expression vector and expressed under the control of an expression regulatory region such as an enhancer or promoter. Next, antibodies can be expressed by transforming host cells with this expression vector. In this regard, appropriate combinations of hosts and expression vectors can be used.

The vectors include, for example, M13 vectors, pUC vectors, pBR322, pBluescript, and pCR-Script. In addition to the above vectors, for example, pGEM-T, pDIRECT, and pT7 can also be used for the subcloning and excision of cDNAs.

When using vectors to produce the antibodies of this invention, expression vectors are particularly useful. When an expression vector is expressed in *E. coli,* for example, it should have the above characteristics in order to be amplified in *E. coli.* Additionally, when *E. coli* such as JM109, DH5 α, HB101, or XL1-Blue are used as the host cell, the vector preferably has a promoter, for example, a lacZ promoter (Ward *et al*. (1989) Nature 341:544-546; (1992) FASEB J. 6:2422-2427), araB promoter (Better *et al*. (1988) Science 240:1041-1043), or T7 promoter, to allow efficient expression of the desired gene in *E. coli*. Other examples of the vectors include pGEX-5X-1 (Pharmacia), "QIAexpress system" (QIAGEN), pEGFP, and pET (where BL21, a strain expressing T7 RNA polymerase, is preferably used as the host).

Furthermore, the vector may comprise a signal sequence for polypeptide secretion. When producing proteins into the periplasm of *E. coli*, the pelB signal sequence (Lei, S. P. *et al*. J. Bacteriol. 169:4379 (1987)) may be used as a signal sequence for protein secretion. For example, calcium chloride methods or electroporation methods may be used to introduce the vector into a host cell.

In addition to E. coli, expression vectors derived from mammals (e.g., pCDNA3 (Invitrogen), pEGF-BOS (Nucleic Acids Res. (1990) 18(17):5322), pEF, pCDM8), insect cells (e.g., "Bac-to-Bac baculovirus expression system" (GIBCO-BRL), pBacPAK8), plants (e.g., pMH1, pMH2), animal viruses (e.g., pHSV, pMV, pAdexLcw), retroviruses (e.g., pZIPneo), yeasts (e.g., "Pichia Expression Kit" (Invitrogen), pNV11, SP-Q01), and *Bacillus subtilis* (e.g., pPL608, pKTH50) may also be used as a vector for producing a polypeptide of the present invention.

In order to express proteins in animal cells, such as CHO, COS, and NIH3T3 cells, the vector preferably has a promoter necessary for expression in such cells, for example, an SV40 promoter (Mulligan *et al.* (1979) Nature 277:108), MMLV-LTR promoter, EF1αpromoter (Mizushima *et al.* (1990) Nucleic Acids Res. 18:5322), CMV promoter, etc.). It is even more preferable that the vector also carries a marker gene for selecting transformants (for example, a drug-resistance gene enabling selection by a drug such as neomycin or G418). Examples of vectors with such characteristics include pMAM, pDR2, pBK-RSV, pBK-CMV, pOPRSV, pOP 13, and such.

In addition, to stably express a gene and amplify the gene copy number in cells, CHO cells with a defective nucleic acid synthesis pathway can be introduced with a vector containing a DHFR gene (for example, pCHOI) to compensate for the defect, and the copy number may be amplified using methotrexate (MTX). Alternatively, a COS cell, which carries an SV40 T antigen-expressing gene on its chromosome, can be transformed with a vector containing the SV40 replication origin (for example, pcD) for transient gene expression. The replication origin may be derived from polyoma viruses, adenoviruses, bovine papilloma viruses (BPV), and such. Furthermore, to increase the gene copy number in host cells, the expression vector may contain, as a selection marker, an aminoglycoside transferase (APH) gene, thymidine kinase (TK) gene, *E. coli* xanthine guanine phosphoribosyl transferase (Ecogpt) gene, dihydrofolate reductase (dhfr) gene, and such.

Methods for expressing the DNAs of this invention in the bodies of animals include methods of incorporating the DNAs of this invention into appropriate vectors and introducing them into living bodies by, for example, a retrovirus method, liposome method, cationic liposome method, or adenovirus method. The vectors used include adenovirus vectors (for example, pAdexlcw), and retrovirus vectors (for example, pZIPneo), but are not limited thereto. General genetic manipulations such as inserting the DNAs of this invention into vectors can be performed according to conventional methods (Molecular Cloning, 5.61-5.63). Administration to living bodies can be carried out by *ex vivo* or *in vivo* methods.

Furthermore, the present invention provides host cells into which a vector of this invention is introduced. The host cells into which a vector of this invention is introduced are not particularly limited; for example, E. coli and various animal cells are available for this purpose. The host cells of this invention may be used, for example, as production systems to produce and express the antibodies of the present invention. *In vitro* and *in vivo* production systems are available for polypeptide production systems. Production systems that use eukaryotic cells or prokaryotic cells are examples of *in vitro* production systems.

Eukaryotic cells that can be used include, for example, animal cells, plant cells, and fungal cells. Known animal cells include mammalian cells, for example, CHO (J. Exp. Med. (1995)108, 945), COS, 3T3, myeloma, BHK (baby hamster kidney), HeLa, Vero, amphibian cells such as *Xenopus laevis* oocytes (Valle, *et al*. (1981) Nature 291, 358-340), or insect cells (e.g., Sf9, Sf21, and Tn5). CHO cells in which the DHFR gene has been deleted, such as dhfr-CHO (Proc. Natl. Acad. Sci. USA (1980) 77, 4216-4220) and CHO K-1 (Proc. Natl. Acad. Sci. USA (1968) 60, 1275), are particularly preferable for use as CHO cells. Of the animal cells, CHO cells are particularly favorable for large-scale expression. Vectors can be introduced into a host cell by, for example, calcium phosphate methods, DEAE-dextran methods, methods using cationic liposome DOTAP (Boehringer-Mannheim), electroporation methods, lipofection methods, etc.

Plant cells include, for example, Nicotiana tabacum-derived cells known as polypeptide production systems. Calluses may be cultured from these cells. Known fungal cells include yeast cells, for example, the genus Saccharomyces, such as Saccharomyces cerevisiae; and filamentous fungi, for example, the genus Aspergillus such as Aspergillus niger.

Bacterial cells can be used in prokaryotic production systems. Examples of bacterial cells include E. coli (for example, JM109, DH5α, HB101 and such); and Bacillus subtilis.

Antibodies can be obtained by transforming the cells with a polynucleotide of interest, then culturing these transformants *in vitro.* Transformants can be cultured using known methods. For example, DMEM, MEM, RPMI 1640, or IMDM may be used as a culture medium for animal cells, and may be used with or without serum supplements such as fetal calf serum (FCS). Serum-free cultures are also acceptable. The preferred pH is about 6 to 8 over the course of culturing. Incubation is typically carried out at about 30 to 40°C for about 15 to 200 hours. Medium is exchanged, aerated, or agitated, as necessary.

On the other hand, production systems using animal or plant hosts may be used as systems for producing polypeptides *in vivo.* For example, a DNA of interest may be introduced into an animal or plant, and the polypeptide produced in the body of the animal or plant is then recovered. The "hosts" of the present invention include such animals and plants.

When using animals, there are production systems using mammals or insects. Mammals such as goats, pigs, sheep, mice, and cattle may be used (Vicki Glaser SPECTRUM Biotechnology Applications (1993)). Alternatively, the mammals may be transgenic animals.

For example, a DNA of interest may be prepared as a fusion gene with a gene encoding a polypeptide specifically produced in milk, such as the goat β-casein gene. DNA fragments containing the fusion gene are injected into goat embryos, which are then introduced back to female goats. The desired antibody can then be obtained from milk produced by the transgenic goats, which are bom from the goats that received the embryos, or from their offspring. Appropriate hormones may be administered to increase the volume of milk containing the polypeptide produced by the transgenic goats (Ebert, K.M. *et al*., Bio/Technology 12, 699-702 (1994)).

Insects, such as silkworms, may also be used. Baculoviruses carrying a DNA of interest can be used to infect silkworms, and the antibody of interest can be obtained from their body fluids (Susumu, M. *et al*., Nature 315, 592-594 (1985)).

When using plants, tobacco can be used, for example. When tobacco is used, a DNA of interest may be inserted into a plant expression vector, for example, pMON 530, and then the vector may be introduced into a bacterium, such as Agrobacterium tumefaciens. The bacteria are then used to infect tobacco, such as Nicotiana tabacum, and the desired polypeptides are recovered from the leaves (Julian K.-C. Ma *et al*., Eur. J. Immunol. 24, 131-138 (1994)).

The resulting antibodies of this invention may be isolated from the inside or outside (such as the medium) of host cells, and purified as substantially pure and homogenous antibodies. Any standard method for isolating and purifying antibodies may be used, and methods are not limited to any specific method. Antibodies may be isolated and purified by selecting an appropriate combination of, for example, chromatographic columns, filtration, ultrafiltration, salting out, solvent precipitation, solvent extraction, distillation, immunoprecipitation, SDS-polyacrylamide gel electrophoresis, isoelectric focusing, dialysis, recrystallization, and others.

Chromatography includes, for example, affinity chromatography, ion exchange chromatography, hydrophobic chromatography, gel filtration, reverse-phase chromatography, and adsorption chromatography (Strategies for Protein Purification and Characterization: A Laboratory Course Manual. Ed Daniel R. Marshak *et al*., Cold Spring Harbor Laboratory Press, 1996). These chromatographies can be carried out using liquid phase chromatographies such as HPLC and FPLC. The present invention also includes antibodies that are highly purified using these purification methods.

In the present invention, the antigen-binding activity of antibodies (Antibodies: A Laboratory Manual. Ed Harlow, David Lane, Cold Spring Harbor Laboratory, 1988) can be measured using well-known techniques. For example, ELISA (enzyme linked immunosorbent assay), EIA (enzyme immunoassay), RIA (radioimmunoassay), or fluoroimmunoassay may be used.

In the present invention, whether or not the antibodies of this invention induce apoptosis in tumor cells can be determined from whether cell death is induced in Daudi cells or Raji cells, as in the Examples.

Furthermore, the present invention provides apoptosis-inducing agents or antitumor agents that comprise minibodies of this invention as active ingredients. Activities of the minibodies in this invention are considered to have a particularly large effect on lymphoma or leukemic cells, therefore, they are considered to be particularly effective for treatment and prevention of tumors such as cancer (particularly blood tumors). When using anti-CD22 antibodies whose molecular weight has not been reduced as active ingredients, they are preferably cross-linked with an anti-IgG antibody or such.

The above-mentioned antibodies can also be used as conjugate antibodies, after linking to various reagents. Examples of such reagents include chemotherapy reagents, radioactive substances, and toxins. Such conjugate antibodies can be produced by known methods (US 5057313, and US 5156840).

The above-mentioned pharmaceutical agents can be directly administered to patients, or administered as pharmaceutical compositions formulated by known pharmaceutical methods. For example, they may be administered orally, as tablets, capsules, elixirs, or microcapsules, sugar-coated as necessary; or parenterally, in the form of injections of sterile solution or suspensions prepared with water or other pharmaceutically acceptable liquids. For example, they may be formulated by appropriately combining them with pharmaceutically acceptable carriers or media, more specifically, sterilized water or physiological saline solutions, vegetable oils, emulsifiers, suspending agents, surfactants, stabilizers, flavoring agents, excipients, vehicles, preservatives, binding agents, and such, and mixing them at a unit dosage form required for generally accepted pharmaceutical practice. The amount of active ingredient in the formulation is such that appropriate doses within indicated ranges are achieved.

Additives that can be mixed into tablets and capsules include, for example, binding agents such as gelatin, cornstarch, tragacanth gum, and gum arabic; excipients such as crystalline cellulose; swelling agents such as cornstarch, gelatin, alginic acid; lubricants such as magnesium stearate; sweeteners such as sucrose, lactose, or saccharine; and flavoring agents such as peppermint and *Gaultheria adenothrix* oils, or cherry. When the unit dosage form is a capsule, liquid carriers such as oils and fats can be further included in the above-indicated materials. Sterile compositions to be injected can be formulated using a vehicle such as distilled water for injection, according to standard protocols.

Aqueous solutions for injection include, for example, physiological saline and isotonic solutions comprising glucose or other adjunctive agents such as D-sorbitol, D-mannose, D-mannitol, and sodium chloride. They may also be combined with appropriate solubilizing agents, such as alcohol, and specifically, ethanol, polyalcohol such as propylene glycol or polyethylene glycol, or non-ionic detergent such as polysorbate 80™ or HCO-50, as necessary.

Oil solutions include sesame oils and soybean oils, and can be combined with solubilizing agents such as benzyl benzoate or benzyl alcohol. Injection solutions may also be formulated with buffers, for example, phosphate buffers or sodium acetate buffers; analgesics, for example, procaine hydrochloride; stabilizers, for example, benzyl alcohol or phenol; or anti-oxidants. The prepared injections are typically aliquoted into appropriate ampules.

Administration to patients may be performed, for example by intra-arterial injection, intravenous injection, or subcutaneous injection, alternatively by intranasal, transbronchial, intramuscular, transdermal, or oral administration using methods well known to those skilled in the art. Doses vary depending on the body weight and age of the patient, method of administration and such; nevertheless, those skilled in the art can appropriately select suitable doses. Furthermore, if a compound can be encoded by a DNA, the DNA may be incorporated into a gene therapy vector to carry out gene therapy. Doses and administration methods vary depending on the body weight, age, and symptoms of patients, but, again, they can be appropriately selected by those skilled in the art.

A single dose of a pharmaceutical agent of this invention varies depending on the target of administration, the target organ, symptoms, and administration method. However, an ordinary adult dose (presuming a body weight of 60 kg) in the form of an injection is approximately 0.1 to 1000 mg, preferably approximately 1.0 to 50 mg, and more preferably approximately 1.0 to 20 mg per day, for example.

When administered parenterally, a single dose varies depending on the target of administration, the target organ, symptoms, and administration method, but in the form of an injection, for example, a single dose of approximately 0.01 to 30 mg, preferably approximately 0.1 to 20 mg, and more preferably approximately 0.1 to 10 mg per day may be advantageously administered intravenously to an ordinary adult (presuming a body weight of 60 kg). For other animals, a converted amount based on the amount for a body weight of 60 kg, or a converted amount based on the amount for a body surface area can be administered.

### Brief Description of the Drawings

Fig. 1 shows the nucleotide sequence and amino acid sequence of an LL2 diabody.
Fig. 2 shows the nucleotide sequence and amino acid sequence of an RFB4 diabody.

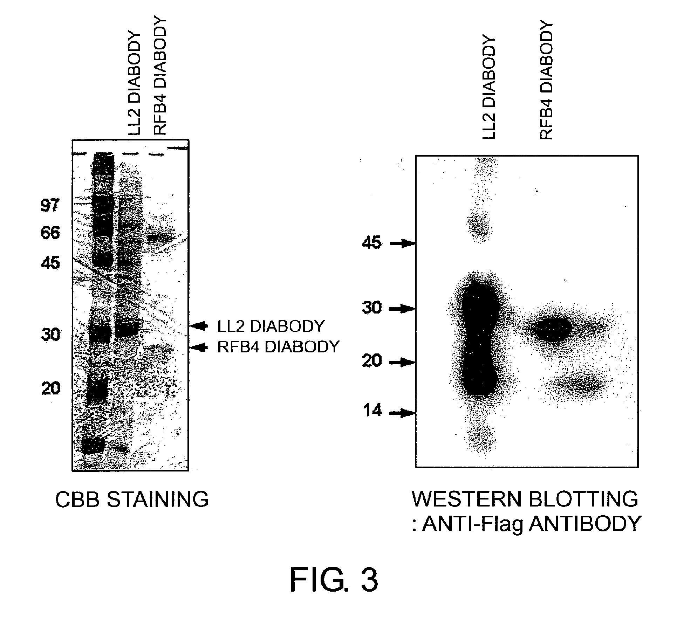

Fig. 3 is a set of photographs showing diabody purity. Each of the purified diabodies was subjected to SDS-PAGE analysis, and then analyzed by CBB staining or Western blotting using anti-Flag antibody. As a result, two diabodies were found to be purified, although not completely.

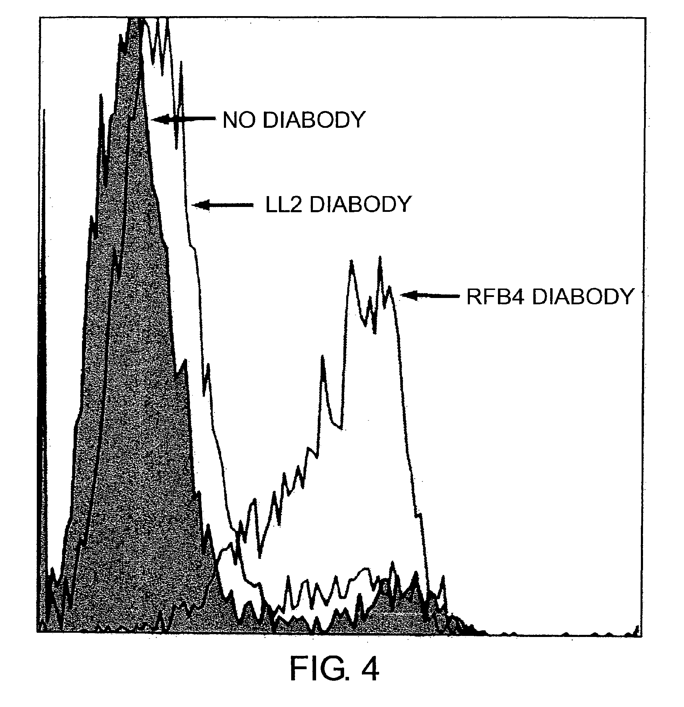

Fig. 4 shows the binding activity of each of the diabodies to Raji cells. The ability of each of the purified diabodies to bind to Raji cells was analyzed. As a result, both diabodies were found to bind to Raji cells. Binding activity was stronger for RFB4 diabody than for LL2 diabody. However, since LL2 antibody is reported to have high activity of internalizing into cells, most of the LL2 diabodies are predicted to have transferred into cells after binding to the cells.

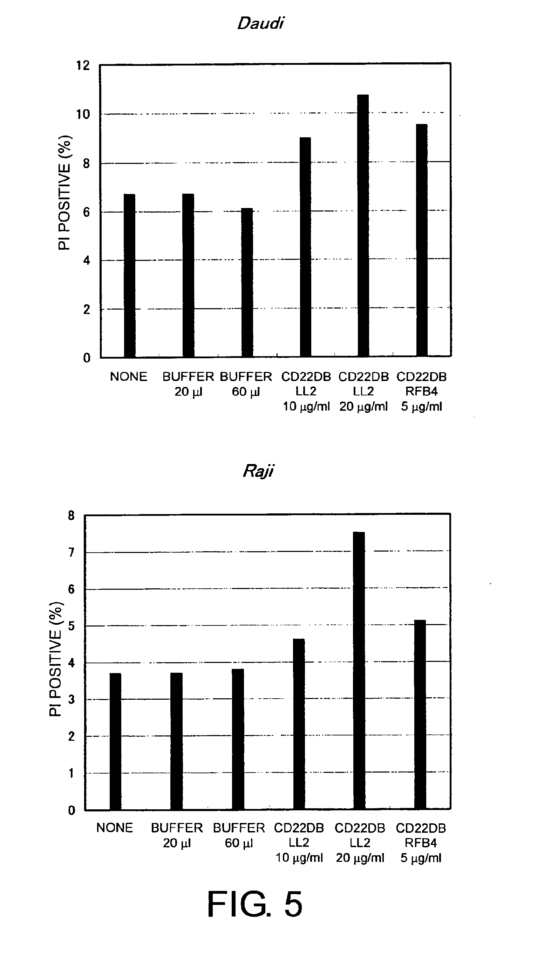

Fig. 5 shows the result of analyzing the cytotoxicity of each diabody. The cytotoxicity of the CD22 diabodies was measured against two types of B-lymphoma cell lines, Daudi and Raji, which are known to strongly express CD22. Each of the diabodies was added to the cells at different concentrations (as indicated in the figure), and 20 hours later, the cells were stained with PI to measure the percentages of dead cells. As a result, both CD22 diabodies were confirmed to induce cell death in B-lymphoma cell lines. These results proved that converted diabodies originated from anti-CD22 antibody can induce cell death in B-lymphoma cell lines by themselves.

### Best Mode for Carrying Out the Invention

Herein below, the present invention is specifically described using Examples, but it is not to be construed as being limited thereto.

### [Example 1] Production of CD22 diabody-expressing vectors

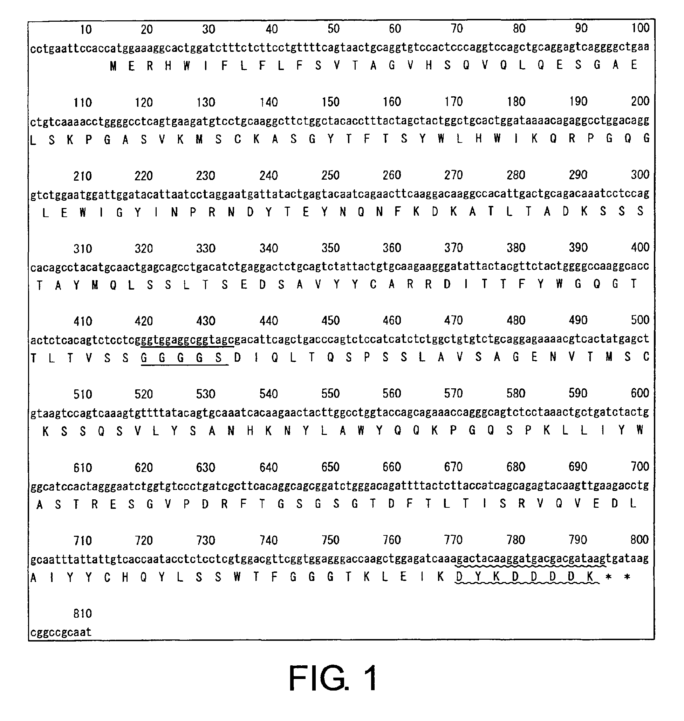

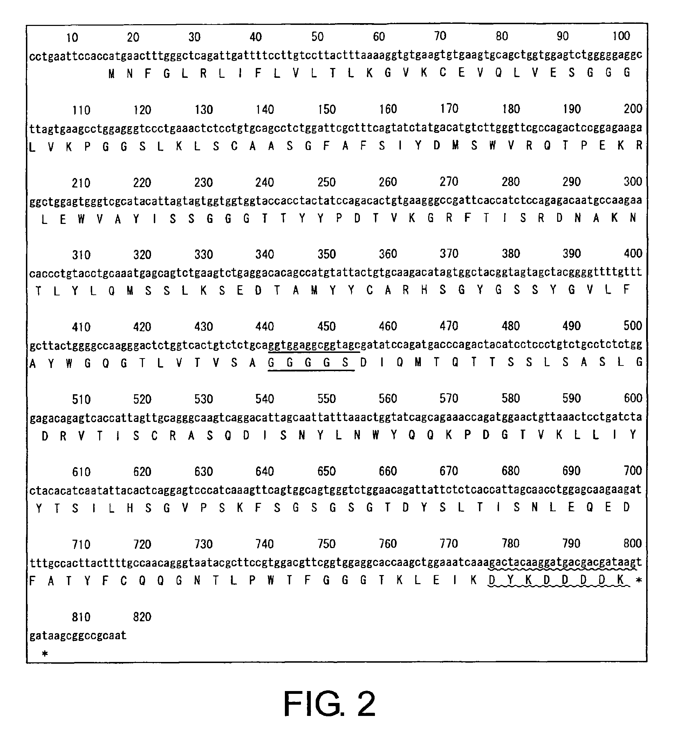

Based on previously known sequence information for two types of anti-CD22 antibodies, specifically LL2 (Patent No. 3053873) and RFB4 (JP 2002501488-A), nucleotide sequences for each of the CD22 diabodies, in which the heavy-chain and light-chain variable regions are linked through a 5-mer linker, were designed (LL2 diabody and RFB4 diabody). Each of the diabody sequences are shown in Fig. 1 (SEQ ID NOs: 1 and 2) and Fig. 2 (SEQ ID NOs: 3 and 4) (linkers are indicated by underlines, and Flag-tags are indicated by wavy lines).

To synthesize cDNAs encoding the designed LL2 diabody and RFB4 diabody, twelve types of oligo-DNAs were produced for each of the diabodies (Espec Oligo Service). The sequences of the synthetic DNAs used are shown in SEQ ID NOs: 13 to 36. cDNAs encoding the diabodies were synthesized as described below. First, two of the oligo-DNAs each were mixed in appropriate combinations. Each mixture was subjected to an annealing and then an elongation reaction in a tube, to produce appropriately 150 bp of DNA fragments. Then, by repeating the recombination reaction several times among the obtained DNA fragments, approximately 800 bp of cDNAs were ultimately synthesized.

Each of the synthesized cDNAs was digested with EcoRI-NotI, and inserted into the EcoRI-NotI gap of the animal cell expression vector, pCXND3. The nucleotide sequences of the vectors were confirmed to complete the construction of an LL2 diabody expression vector (pCXND3-LL2DB) and RFB4 diabody expression vector (pCXND3-RFB4DB).

### [Example 2] Purification of CD22 diabodies

### (1) Establishment of cell lines expressing LL2 diabody and collection of culture supernatant

20 µg of pCXND3-LL2DB, linearized by cleaving with PvuI, was introduced into DG44 cells by electroporation, as described below. After washing DG44 cells twice with ice-cold PBS, they were suspended in PBS to a density of 1x 10⁷ cells/ml. 20 µg of the above-mentioned plasmid was mixed into the suspension, and subjected to electroporation (1.5 kV, 25 µFD). The cells were appropriately diluted, plated onto a 96-well plate, and cultured in the presence of G418 (GIBCO) at a final concentration of 500 µg/ml. Approximately 30 cell clones were selected from wells in which colonies had grown, and diabody expression levels in the culture supernatants were investigated by Western blotting. Clones showing diabody expression were expanded to use as LL2 diabody-overproducing cell lines. A confluent LL2 diabody-overproducing cell line in a T-175 flask was transferred to two roller bottles (250 ml of CHO-S-SFMII media (GIBCO)), and the culture supernatant was collected five days later. After removing the dead cells by centrifugation, the culture supernatant was passed through a 0.45 µm filter and used for LL2 diabody purification.

### (2) Transient expression of RFB4 diabodies in cos7, and collection of culture supernatant

20 µg of pCXND3-RFB4DB was introduced into COS7 cells by an electroporation method, as described below. After washing the COS7 cells twice with ice-cold PBS, they were suspended in PBS to a density of 1x 10⁷ cells/ml. 20 µg of the above-mentioned plasmid was mixed into the suspension, and subjected to electroporation (220 V, 950 µFD). Then, all cells were placed in to three T-225 flasks (DMEM + 10% FCS), and the culture supernatant was collected three days later. After removing the dead cells by centrifugation, the culture supernatant was passed through a 0.45 µm filter and used for RFB42 diabody purification.

### (3) Purification of diabodies

Purification of diabodies was performed as follows: Anti-Flag M2 Agarose (SIGMA) was added to each of the collected culture supernatants, and mixed overnight at 4°C to adhere the diabodies. Anti-Flag M2 Agarose was collected by centrifugation, and washed several times with PBS, and then the diabodies were eluted using an elution buffer (100 mM Glycine pH3.5, 0.01 % Tween 20). The collected samples were immediately neutralized with Tris-HCl (pH8.0) such that the final concentration was 25 mM. The samples were then concentrated, and the buffer was changed to PBS containing 0.01% of Tween 20. A portion of the collected samples was subjected to SDS electrophoresis, and then Western blotting using the anti-FLAG antibody and Coomassie staining were carried out to confirm that the target protein was purified.

### [Example 3] Confirmation of binding of CD22 diabodies to lymphoma cells

Purified LL2 diabody and RFB4 diabody were added to cells of the B-lymphoma cell line, Raji, in PBS containing 2% FCS and 0.02% NaN₃ such that the final concentrations were 20 µg/mL and 8 µg/mL, respectively. After reacting on ice for one hour, anti-Flag M2 antibody was added to the mixture, and then reacted on ice for another one hour. The cells were washed and reacted with FITC-anti-mouse IgG on ice for 30 minutes. Diabody binding to the cell surface was measured by flow cytometry (EPICS ELITE, COULTER).

### [Example 4] Analysis of the lymphoma cell death-inducing activities of CD22 diabodies

B-lymphoma cell lines, Raji and Daudi, were plated onto 24-well plates at a density of 2x - 5x 10⁵ cells/well. Purified LL2 diabody or RFB4 diabody were added to each of the wells, which were then cultured at 37°C. The cells were collected 20 hours later, and dead cells were labeled by adding PI and then reacted at room temperature for 15 minutes. Thereafter, the percentage of stained dead cells was measured by flow cytometry (EPICS ELITE, COULTER).

### Industrial Applicability

This invention provides minibodies with high specific activities. By using these minibodies, adequate drug efficacy can be expected even with a short half-life. The minibodies of the present invention are further expected to be able to separate drug efficacy from toxicity. In addition, since overall cost is reduced, including reducing clinical dose and production cost, economical problems of concern in the development of antibody pharmaceuticals are also expected to improve.

## Claims

1. A minibody that recognizes CD22.

2. The minibody of claim 1, wherein the minibody is a diabody.

3. A minibody of any one of (a) to (f):
(a) a minibody comprising the amino acid sequence of SEQ ID NO: 1 or 3;
(b) a minibody functionally equivalent to the minibody of (a), and comprising the amino acid sequence of SEQ ID NO: 1 or 3 wherein one or more amino acids are substituted, inserted, deleted, and/or added;
(c) a minibody comprising the amino acid sequences of a CDR of SEQ ID NOs: 5 and 7;
(d) a minibody functionally equivalent to the minibody of (c), and comprising the amino acid sequence of a CDR of SEQ ID NOs: 5 and 7 wherein one or more amino acids are substituted, inserted, deleted, and/or added;
(e) a minibody comprising the amino acid sequences of a CDR of SEQ ID NOs: 9 and 11; and
(f) a minibody functionally equivalent to the minibody of (c), and comprising the amino acid sequence of a CDR of SEQ ID NOs: 9 and 11 wherein one or more amino acids are substituted, inserted, deleted, and/or added.

4. A method for producing a CD22-recognizing antibody with increased activity by converting a CD22-recognizing antibody to a low-molecular-weight antibody.

5. The method of claim 4, wherein the conversion is conversion to a diabody.

6. The method of claim 4 or 5, wherein the activity is an apoptosis-inducing activity.

7. An apoptosis-inducing agent comprising the minibody of any one of claims 1 to 3, or the minibody produced by the method of any one of claims 4 to 6, as an active ingredient.

8. The apoptosis-inducing agent of claim 7 that induces tumor cell apoptosis.

9. The apoptosis-inducing agent of claim 8, wherein the tumor cell is a lymphoma or leukemic cell.

10. An antitumor agent comprising the minibody of any one of claims 1 to 3, or the minibody produced by the method of any one of claims 4 to 6, as an active ingredient.

11. The antitumor agent of claim 10, wherein the tumor is a blood tumor.

12. The apoptosis-inducing agent of any one of claims 7 to 9, wherein the antibody is a diabody.

13. The antitumor agent of claim 10 or 11, wherein the antibody is a diabody.
